# EUROPÄISCHE PATENTSCHRIFT

(11) **EP 0 538 192 B1**
(45) Veröffentlichungstag und Bekanntmachung des Hinweises auf die Patenterteilung: **23.04.1997**
(21) Anmeldenummer: 92810766.3
(22) Anmeldetag: 08.10.1992
(51) Int. Cl.: C07J 73/00, A61K 31/58

(54) **Neue 17beta-substituierte Aza-androstan-Derivate**
New 17-beta-substituted aza-androstanderivatives
Dérivés nouveaux d'une aza-androstan substituée en position 17-bêta

(30) Priorität: 09.10.1991 CH 2978/91
(43) Veröffentlichungstag der Anmeldung: 21.04.1993
(73) Patentinhaber: Novartis AG, 4058 Basel (CH)
(72) Erfinder: Biollaz, Michel, Dr., CH-4102 Binningen (CH)

(56) Entgegenhaltungen:
- EP-A- 0 271 220
- JOURNAL OF THE AMERICAN CHEMICAL SOCIETY, Band 110, Mai-Juni 1988, A. BHATTARCHAYA et al., Seiten 3318-3319
- JOURNAL OF MEDICINAL CHEMISTRY, Band 29, Nr. 7, Juli 1986, G.M. RASMUSSEN et al., Seiten 2298-2315

## Beschreibung

Die Erfindung betrifft neue 17β-substituierte Aza-androstan-Derivate der Formel 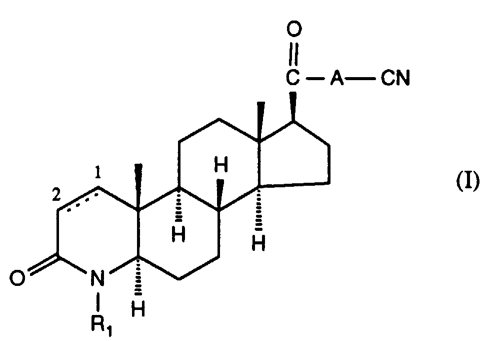 worin die Kohlenstoffatome 1 und 2 durch eine Einfachbindung oder eine Doppelbindung verbunden sind, R₁ Wasserstoff, Methyl oder Ethyl bedeutet, und A für eine Gruppe der Formel -N(-R₂)-X-, worin R₂ Wasserstoff oder C₁-C₄-Alkyl, und X C₁-C₁₂-Alkylen oder C₃-C₆- Cycloalkyliden bedeutet; eine Gruppe der Formel -N(-R₂)-Y-Phe-, worin R₂ die oben genannte Bedeutung hat, Y eine direkte Bindung oder C₁-C₆-Alkylen ist und Phe einen gegebenenfalls substituierten Phenylenrest bezeichnet; eine Gruppe der Formel -O-X-, worin X die oben genannte Bedeutung hat, oder eine Gruppe -O-Y-Phe- steht, worin Y und Phe die oben genannten Bedeutungen haben.

Die Erfindung betrifft auch Verfahren zur Herstellung der oben genannten Verbindungen, sowie diese Verbindungen enthaltende pharmazeutische Zusammensetzungen und Verfahren zu ihrer Herstellung; ferner auch die therapeutische Anwendung dieser Verbindungen und diese enthaltender pharmazeutischen Zusammensetzungen bei Warmblütern einschliesslich des Menschen.

Die vor- und nachstehend verwendeten Definitionen haben im Rahmen der vorliegenden Beschreibung vorzugsweise die folgenden Bedeutungen:

C₁-C₄- Alkyl ist entsprechendes geradkettiges oder verzweigtes Alkyl und bedeutet z.B. n-Propyl, n-Butyl, Isopropyl, tert. Butyl und insbesondere Methyl und Ethyl.

C₁-C₁₂-Alkylen als Rest X kann linear oder beliebig verzweigt sein, wobei die zwei freien Valenzen von zwei verschiedenen C-Atomen oder vom selben C-Atom ausgehen können. Bevorzugt sind sowohl lineare C₂-C₆-Alkylenreste, z.B. Tri- bis Hexamethylen, insbesondere Pentamethylen, und Ethylen, als auch verzweigte C₃-C₆-Alkylenreste, z.B. 2-Methyl-1,2-propylen und 1,1-Dimethylethylen, die die freien Valenzen an zwei verschiedenen C-Atomen haben. Hervorzuheben sind auch lineare oder höchstens einmal verzweigte Alkylenreste, deren beide freien Valenzen von demselben C-Atom ausgehen, z.B. lineare oder einmal verzweigte C₁-C₆-Alkylidenreste, wie in erster Linie Methylen, aber auch Ethyliden, 1,1-Propyliden oder 2,2-Propyliden. In Übereinstimmung mit der obigen Definition des Begriffes Alkylen können z.B. 1,1-Alkylidenreste auch als 1,1-Alkylenreste bezeichnet werden.

C₃-C₆-Cycloalkyliden ist 1,1-Cyclopropyliden, 1,1-Cyclobutyliden, 1,1-Cyclopentyliden und 1,1-Cyclohexyliden.

C₁-C₆-Alkylen (Y) kann linear oder verzweigt sein. Bevorzugt ist lineares C₁-C₄-Alkylen, z.B. Methylen, Ethylen, Trimethylen und Tetramethylen.

Phenylen ist ortho (o-), meta (m-) und insbesondere para (p-) Phenylen und kann abgesehen von der Cyanogruppe - unsubstituiert sein oder einen, zwei oder drei weitere Substituenten ausgewählt aus der Gruppe Halogen, C₁-C₄-Alkyl, C₁-C₄-Alkoxy, Hydroxy, Nitro, Cyano und C₁-C₄-Alkoxycarbonyl tragen.

Halogen ist z.B. Fluor oder Chlor.

C₁-C₄-Alkoxy ist z.B. Propoxy, Butoxy und insbesondere Methoxy und Ethoxy.

C₁-C₄-Alkoxycarbonyl ist beispielsweise Propoxycarbonyl, tert. Butoxycarbonyl und insbesondere Methoxycarbonyl und Ethoxycarbonyl.

Die Verbindungen der Formel I weisen wertvolle pharmakologische Eigenschaften auf. Insbesondere sind sie potente Inhibitoren des Enzyms 5α-Reduktase, welches für die Umwandlung des beim Manne vorwiegend zirkulierenden Androgens Testosteron in das noch stärker wirkende 5α-Dihydrotestosteron verantwortlich ist. Bei der benignen Prostata-Hypertrophie wird 5α-Dihydrotestosteron in der Prostata in erhöhter Konzentration nachgewiesen und deshalb für die Hypertrophie verantwortlich gemacht. Es wurde jetzt gefunden, dass die erfindungsgemässen Verbindungen der Formel I eine starke Hemmwirkung auf das Enzym 5α-Reduktase ausüben.

Die erfindungsgemässen Verbindungen der Formel I sind daher bei Warmblütern (Mensch und Tier) geeignet für die therapeutische Behandlung von benigner Prostata-Hypertrophie und anderen Erkrankungen und Zuständen, die auf eine Reduktion der physiologischen 5α-Dihydrotestosteron-Menge günstig ansprechen, wie Prostatakarzinom, Seborrhoe, Akne vulgaris, weiblicher Hirsutismus und dergleichen. Die neuen Verbindungen können als enteral, z.B. oral, topisch oder parenteral applizierbare 5α-Reduktase-Hemmer, z.B. in Form von entsprechenden pharmazeutischen Präparaten, Verwendung finden.

Die Erfindung betrifft insbesondere Verbindungen der Formel I, worin die Kohlenstoffatome 1 und 2 durch eine Einfachbindung oder eine Doppelbindung verbunden sind, R₁ Wasserstoff, Methyl oder Ethyl bedeutet, und A für eine Gruppe der Formel -N(-R₂)-X-, worin R₂ Wasserstoff oder C₁-C₄-Alkyl, insbesondere Methyl, und X geradkettiges oder verzweigtes C₁-C₁₂-Alkylen, insbesondere C₁-C₆-Alkylen, wie Ethylen, Pentamethylen, 2-Methyl-1,2-propylen oder 2,2-Propyliden, oder C₃-C₆- Cycloalkyliden, z.B. 1,1-Cyclopropyliden, bedeutet; eine Gruppe der Formel -N(-R₂)-Y-Phe-, worin R₂ die oben genannte Bedeutung hat, Y eine direkte Bindung oder C₁-C₆-Alkylen, insbesondere C₁-C₄-Alkylen, z.B. Methylen, ist, und Phe einen Phenylenrest bezeichnet, der gegebenenfalls durch Halogen, z.B. Chlor, C₁-C₄-Alkyl, z.B. Methyl, C₁-C₄-Alkoxy, wie Methoxy, Hydroxy, Nitro, Cyano und/oder C₁-C₄-Alkoxycarbonyl, z.B. Methoxycarbonyl, substituiert ist; eine Gruppe der Formel -O-X-, worin X die oben genannte Bedeutung hat, oder eine Gruppe -O-Y-Phe steht, wobei Y und Phe die oben genannten Bedeutungen haben.

Die Erfindung betrifft in erster Linie Verbindungen der Formel I, worin die Kohlenstoffatome 1 und 2 durch eine Einfachbindung oder eine Doppelbindung verbunden sind, R₁ Wasserstoff oder Methyl bedeutet, und A für eine Gruppe der Formel -N(-R₂)-X-, worin R₂ Wasserstoff und X geradkettiges oder verzweigtes C₁-C₆ Alkylen, insbesondere Ethylen, Pentamethylen, 2-Methyl-1,2-propylen oder 2,2-Propyliden bedeutet; eine Gruppe der Formel -N(-R₂)-Y-Phe-, worin R₂ die oben genannte Bedeutung hat, Y eine direkte Bindung oder C₁-C₄-Alkylen, z.B. Methylen, ist, und Phe einen Phenylenrest bezeichnet, der gegebenenfalls durch Halogen, z.B. Chlor, C₁-C₄-Alkyl, z.B. Methyl, C₁-C₄-Alkoxy, wie Methoxy, Hydroxy, Nitro, Cyano oder C₁-C₄-Alkoxycarbonyl, z.B. Methoxycarbonyl, substituiert ist; eine Gruppe der Formel -O-X-, worin X die oben genannte Bedeutung hat, oder eine Gruppe -O-Y-Phe- steht, wobei Y und Phe die oben genannten Bedeutungen haben.

Besonders hervorzuheben sind Verbindungen der Formel I, worin die Kohlenstoffatome 1 und 2 durch eine Doppelbindung verbunden sind, R₁ Wasserstoff bedeutet, und A für eine Gruppe der Formel -N(-R₂)-X-, worin R₂ Wasserstoff und X C₁-C₄ Alkylen, insbesondere 2,2-Propyliden bedeutet; oder eine Gruppe der Formel -N(-R₂)-Y-Phe-, worin R₂ die oben genannte Bedeutung hat, Y eine direkte Bindung ist, und Phe einen p-Phenylenrest bezeichnet.

Die Erfindung betrifft bevorzugt die in den Beispielen beschriebenen Verbindungen der Formel I.

Die Verbindungen der vorliegenden Erfindung werden nach an sich bekannten Verfahren erhalten.

Die erfindungsgemässen Verbindungen der Formel I werden beispielsweise hergestellt, in dem man
(a) eine Verbindung der Formel 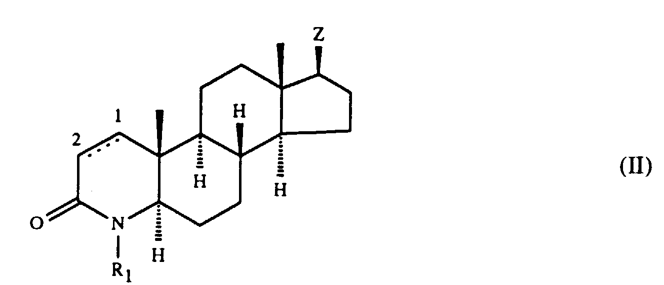 worin die Kohlenstoffatome 1 und 2 durch eine Einfachbindung oder eine Doppelbindung verbunden sind, R₁ die unter Formel I angegebene Bedeutung hat, und Z für Carboxyl oder eine reaktionsfähig aktivierte Carboxylgruppe steht, mit einer Verbindung der Formel H-A₁-CN, worin A₁ für eine Gruppe der Formel -N(-R₂)-X- oder für eine Gruppe der Formel -N(-R₂)-Y-Phe- steht, oder mit einer Verbindung der Formel H-A₂-CN umsetzt, worin A₂ für eine Gruppe der Formel -O-X- oder für eine Gruppe der Formel -O-Y-Phe-steht, worin R₂,X, Y und Phe die unter Formel I angegebenen Bedeutungen haben, oder
(b) in einer Verbindung der Formel 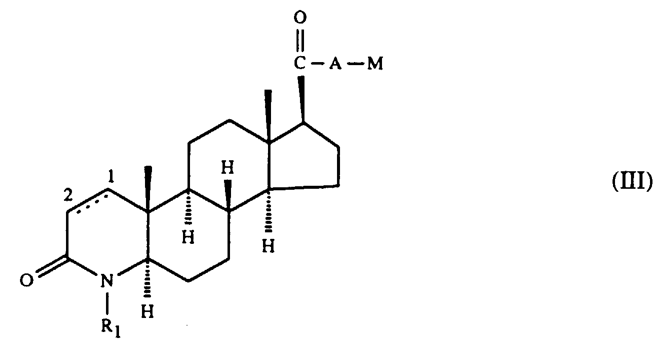 worin die Kohlenstoffatome 1 und 2 durch eine Einfachbindung oder eine Doppelbindung verbunden sind, R₁ und A wie unter Formel I definiert sind, und M einen in Cyano überführbaren Rest bedeutet, den Rest M in Cyano überführt, oder
(c) zur Herstellung einer Verbindung der Formel I, worin die Kohlenstoffatome 1 und 2 durch eine Einfachbindung verbunden sind, und R₁ und A die oben angegebenen Bedeutungen haben, eine Verbindung der Formel 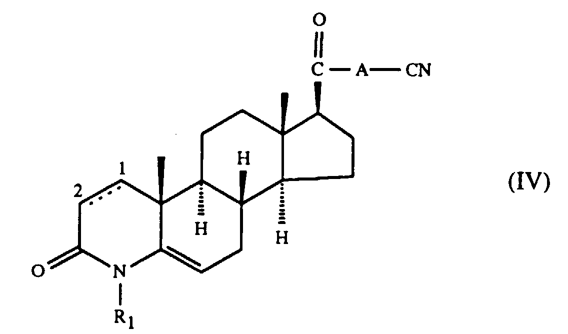 mit einem Reduktionsmittel behandelt, und wenn erwünscht, eine verfahrensgemäss erhältliche Verbindung der Formel I in eine andere Verbindung der Formel I überführt.

Bei dem Verfahren (a) handelt es sich um eine Acylierungsreaktion. Eine Aminoverbindung der Formel H-A₁-CN oder eine Hydroxyverbindung der Formel H-A₂-CN wird mit einer Carbonsäure oder einem reaktionsfähigen Derivat davon unter Ausbildung eines Säureamids (Carboxamid) bzw. eines Esters (Carboxylat) acyliert, wobei die Aktivierung der als Acylierungsmittel verwendeten Carbonsäure auch in Gegenwart der zu acylierenden Verbindung erfolgen kann (siehe z.B. Haslam, E. , Tetrahedron 36, 2409-2433 (1980)).

Als Acylierungsmittel verwendbare Carbonsäurederivate sind in erster Linie reaktionsfähige aktivierte Ester oder reaktionsfähige Anhydride, ferner reaktionsfähige cyclische Amide.

Geeignete aktivierte Ester sind zum Beispiel Ester vom Amidinotyp, wie N,N'-disubstituierte Amidinoester, die man z.B. durch Behandeln der entsprechenden Säure der Formel II, worin Z Carboxyl bedeutet, mit einem geeigneten N,N'-disubstituierten Carbodiimid, z.B. N,N'-Dicyclohexylcarbodiimid erhalten kann (Carbodiimidmethode); Thioester, insbesondere 2-Pyridylthioester, die z.B. bei Umsetzung der entsprechenden Säure mit Triphenylphosphin und 2,2'-Dithio-dipyridin entstehen (Methode der aktivierten Thioester); oder N-Hydroxyester, z.B. Aminoester oder Amidoester, die man beispielsweise durch Behandeln der entsprechenden Säure mit einer N-Hydroxyamino- bzw. N-Hydroxyamido-Verbindung, z.B. N-Hydroxysuccinimid, N-Hydroxypiperidin, N-Hydroxyphtalimid oder 1-Hydroxybenzotriazol, z.B. nach der Carbodiimidmethode, erhalten kann. Zu den bevorzugten Aminoestern zählen Benzotriazol-1-yl-oxyderivate. Letztere entstehen beispielsweise bei Umsetzung der entsprechenden Säure der Formel II, worin Z Carboxyl bedeutet, mit einem geeigneten Benzotriazolderivat, insbesondere Benzotriazol-1-yl-oxy-tris-(dimethylamino)phosphonium-hexafluorphosphat (Castros Reagenz) oder O-(1H-Benzotriazol-1-yl)N,N,N',N'-tetramethyluronium-hexafluorphosphat.

Geeignete Säureanhydride sind insbesondere gemischte Anhydride einer Säure der Formel II, worin Z Carboxy bedeutet, so z.B. Anhydride mit anorganischen Säuren, wie Säurehalogenide, insbesondere Säurechloride, die man beispielsweise durch Behandeln der entsprechenden Säure mit Oxalylchlorid, Thionylchlorid oder 1-Chlor-1-dimethylamino-2-methyl-prop-1-en erhält (Säurechloridmethode).

Geeignete cyclische Amide sind insbesondere Amide mit fünfgliedrigen Diazacyclen aromatischen Charakters, wie Amide mit Imidazolen, z.B. Imidazol, die man z.B. durch Behandeln der entsprechenden Säure mit N,N'-Carbonyldiimidazol erhalten kann (Imidazolid-Methode).

Wie erwähnt können Derivate von Säuren, die als Acylierungsmittel verwendet werden, in situ gebildet werden. So kann man beispielsweise N-Hydroxyester in situ bilden, indem man das Gemisch des zu acylierenden Ausgangsmaterials und der als Acylierungsmittel verwendeten Säure in Gegenwart eines geeigneten Benzotriazolderivats, z.B. Benzotriazol-1-yl-oxy-tris(dimethylamino)-phosphonium-hexafluorphosphat, umsetzt. Ferner kann man Amino- oder Amidoester der als Acylierungsmittel verwendeten Säuren in Gegenwart des zu acylierenden Ausgangsmaterials in situ bilden, indem man das Gemisch der entsprechenden Säure- und Amino-Ausgangsstoffe in Gegenwart eines N,N'-disubstituierten Carbodiimids, z.B. N,N'-Dicyclohexylcarbodiimid, und eines N-Hydroxyamins, insbesondere Hydroxybenzotriazol, oder eines N-Hydroxyamids, z.B. N-Hydroxysuccinimid, gegebenenfalls in Gegenwart einer geeigneten Base, z.B. 4-Dimethylaminopyridin, umsetzt. N,N'-disubstituierte Amidinoester entstehen beispielsweise in situ bei der Umsetzung der zu acylierenden Verbindung mit der als Acylierungsmittel verwendeten Säure in Gegenwart eines geeigneten N,N-disubstituierten Carbodiimids, z.B. N,N'-Dicyclohexylcarbodiimid.

Die Acylierung kann in an sich bekannter Weise durchgeführt werden, üblicherweise bei Temperaturen zwischen dem Gefrierpunkt und dem Siedepunkt des Reaktionsgemisches, wie im Temperaturbereich von etwa - 10 bis etwa + 150°C, vorzugsweise von Raumtemperatur (ca. +20°C) bis etwa +70°C, z.B. in einem geschlossenen Reaktionsgefäss und/oder in der Atmosphäre eines Inertgases, z.B. Stickstoff, in Gegenwart eines geeigneten Lösungsmittels, vorzugsweise eines inerten Lösungsmittels, wie Dimethylformamid (DMF) eines Ethers, z. B. Tetrahydrofuran (THF), oder eines halogenierten, insbesondere chlorierten, aliphatischen Kohlenwasserstoffs, beispielsweise Chloroform oder Methylenchlorid, und gegebenenfalls in Gegenwart eines säurebindenden Mittels, z.B. einer Base. Eine geeignete Base ist z.B. ein Amin, z.B. ein tertiäres Amin, etwa ein Tri-(C₁-C₄)-Alkylamin wie Trimethylamin, Triethylamin oder Ethyldiisopropylamin, oder ein Arylalkylamin, z.B. N,N-Dimethylanilin, oder ein zyklisches tertiäres Amin, z.B. N-C₁-C₄-Alkyl-morpholin, z.B. N-Methylmorpholin, oder eine Base vom Pyridin-Typ, z.B. Pyridin oder Chinolin.

Das Verfahren (b) wird gemäss bekannten Methoden zur Einführung der Cyangruppe durchgeführt.

Reste M in einer Verbindung der Formel III, die in Cyano überführt werden können, sind z.B. Wasserstoff; veresterte Hydroxygruppen, z.B. Halogen, insbesondere Chlor, Brom oder Jod, oder Sulfonyloxy, z.B. Toluolsulfonyloxy, Benzolsulfonyloxy oder Methansulfonyloxy; Carboxy, Carboxy in Form eines funktionellen Derivates, z.B. Aminocarbonyl, C₁-C₄-Alkylaminocarbonyl, z.B. tert.-Butylaminocarbonyl, oder Formyl in Form eines funktionellen Derivates, z.B. Hydroxyiminomethyl.

Verbindungen der Formel I können gemäss Verfahren (b) z.B. durch die folgenden Umsetzungen erhalten werden:

Die Umsetzung einer Verbindung der Formel III, worin M Halogen bedeutet, z.B. Chlor, Brom, Jod, zu einer entsprechenden Verbindung der Formel I wird z.B. unter Verwendung eines Cyanidsalzes, insbesondere von Natrium- oder Kaliumcyanid oder Kupfer(I)cyanid, durchgeführt. Bevorzugt werden dabei hohe Temperaturen, insbesondere die Rückflusstemperatur des Reaktionsgemisches.

Die Umsetzung einer Verbindung der Formel III, worin M Sulfonyloxy, z.B. p-Toluolsulfonyloxy, Benzolsulfonyloxy oder Methansulfonyloxy, bedeutet, zu einer entsprechenden Verbindung der Formel I wird z. B. durch Reaktion mit einem Alkalimetallcyanid , vorzugsweise Natrium- oder Kaliumcyanid, durchgeführt. Bevorzugt werden dabei hohe Temperaturen, insbesondere die Rückflusstemperatur des Reaktionsgemisches.

Die Umsetzung einer Verbindung der Formel III, worin M Carboxy bedeutet, in eine Verbindung der Formel I kann z.B. durch Reaktion mit einem Chlorsulfonylisocyanat in z.B. Dimethylformamid (DMF) nach der Methode von R. Graf in Angew. Chem. 80, 183 (1968) durchgeführt werden.

Die Umsetzung einer Verbindung der Formel III, worin M Carboxy bedeutet, das in Form eines funktionellen Derivates vorliegt, z.B. als Aminocarbonyl oder C₁-C₄-Alkylaminocarbonyl, vorteilhafterweise als tert-Butylaminocarbonyl, in eine Verbindung der Formel I kann z.B. mit einem starken Dehydratisierungsmittel, z.B. Phosphor(V)oxid, Phosphorylchlorid, Thionylchlorid, Tosylchlorid, Mesylchlorid oder Oxalylchlorid, durchgeführt werden. Die Dehydratisierung erfolgt vorzugsweise in einem inerten wasserfreien Lösungsmittel, beispielsweise einem Ether, z.B. Tetrahydrofuran oder Dioxan, DMF, ferner auch Pyridin, bei Raumtemperatur oder etwas erniedrigter oder erhöhter Temperatur, z.B. bei etwa 0°C bis etwa 80°C.

Die Umsetzung einer Verbindung der Formel III, worin M Formyl bedeutet, in eine entsprechende Verbindung der Formel I wird z.B. nach Überführung der Formylgruppe in ein funktionelles Derivat, z.B. Hydroxyiminomethyl (Aldoxim), und Umwandlung dieser Gruppe durch ein Dehydratisierungsmittel in eine Cyangruppe durchgeführt. Die Umsetzung der Formylgruppe in die Hydroxyiminomethylgruppe erfolgt z. B. durch Umsetzung mit einem Salz des Hydroxylamins, vorzugsweise dem Hydrochlorid. Ein geeignetes Dehydratisierungsmittel ist beispielsweise eines der oben genannten, z.B. Phosphor(V)oxid oder Thionylchlorid, das Anhydrid einer organischen Säure, z.B. das Anhydrid einer C₁-C₄-Alkansäure, vorzugsweise Essigsäureanhydrid, Oxalsäurediimidazolid (siehe T. Kitagawa et al., Chem. Pharm. Bull. 33, 4014 (1985)) oder Benzolselenylchlorid (siehe G. Sosnovsky, J.A. Rogh, Z. Naturforsch. B 34, 511 (1979)). Die Dehydratisierung wird in an sich bekannter Weise durchgeführt, beispielsweise in Gegenwart eines geeigneten Lösungsmittels, vorzugsweise eines inerten wasserfreien Lösungsmittels, wie eines Ethers, z.B. Diethylether oder Tetrahydrofuran (THF), eines halogenierten, insbesondere chlorierten, aliphatischen Kohlenwasserstoffs, beispielsweise Chloroform oder Methylenchlorid, oder eines aromatischen Kohlenwasserstoffs, beispielsweise Benzol.

Eine Verbindung der Formel III, worin M Formyl bedeutet, kann auch direkt in das entsprechende Nitril der Formel I umgesetzt werden, z.B. durch Reaktion mit O,N-Bis-(trifluoracetyl)-hydroxylamin in Gegenwart einer Base, z.B. Pyridin, nach der Methode von D.T. Mowry, Chem. Revs. 42, 251 (1948).

Verbindungen der Formel I können gemäss Verfahren (c) durch Reduktion, insbesondere mit Wasserstoff in Gegenwart eines Übergangsmetallkatalysators (katalytische Hydrierung), einer Verbindung der Formel IV erhalten werden. Die katalytische Hydrierung wird beispielsweise mit Palladium oder Platinoxid, gegebenenfalls unter Zusatz von Kohle, bevorzugt in saurem Medium, z.B. in Eisessig, durchgeführt.

Verfahrensgemäss erhältliche Verbindungen der Formel I können in an sich bekannter Weise in andere Verbindungen der Formel I umgewandelt werden.

So können beispielsweise Verbindungen der Formel I, worin R₁ und/oder R₂ für Wasserstoff steht, durch Umsetzen mit einer starken Base, z.B. Natriumhydrid oder Natriumamid, und einem C₁-C₄-Alkylhalogenid in einem wasserfreien inerten Lösungsmittel, z.B. DMF oder THF, in Verbindungen der Formel I umgewandelt werden, worin R₁ Methyl oder Ethyl und R₃ C₁-C₄-Alkyl bedeuten.

1,2-gesättigte Verbindungen der Formel I können in an sich bekannter Weise zu den entsprechenden 1,2-Dehydroderivaten dehydriert werden. Man kann dazu biologische Dehydrierungsverfahren anwenden, z.B. mittels der Mikroorganismen Corynebacterium simplex oder Septomyxa affinis oder ihrer Enzymsysteme dehydrieren, oder mit Selendioxid in einem organischen Lösungsmittel, z.B. tert.Butylalkohol, behandeln. Vorzugsweise dehydriert man jedoch mit 2,3-Dichlor-5,6-dicyan- 1,4-benzochinon (gegebenenfalls in Gegenwart von Bis-(trimethylsilyl)-trifluoracetamid, wobei ein intermediäres O-Trimethylsilylimidat entsteht) oder mit Benzolseleninsäureanhydrid während mehrerer, z.B. 6-24 Stunden, und gegebenenfalls bei Raumtemperatur oder erhöhter Temperatur, z.B. Siedehitze, in organischen Lösungsmitteln, z.B. aromatischen Kohlenwasserstoffen, wie Benzol oder Xylol, niederen aliphatischen Alkoholen, wie Ethanol, Propanol oder tert.Butylalkohol, niederen aliphatischen Ketonen, wie Aceton oder 2-Butanon, aliphatischen Estern, wie Ethylacetat, oder cyclischen Ethern, wie Dioxan oder Tetrahydrofuran.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt oder man das Verfahren auf irgendeiner Stufe abbricht, oder bei denen ein Ausgangsstoff unter den Reaktionsbedingungen gebildet oder in Form eines Derivates, z.B. eines Salzes davon verwendet wird.

Bei den Verfahren der vorliegenden Erfindung werden bekannte oder nach bekannten Methoden erhältliche Ausgangsstoffe verwendet (vgl. G.H. Rasmusson et al., J. Med. Chem. 29, 2298-2315 (1986); J. Am. Chem. Soc. (JACS) 110, 3318 ( 1988)), vorzugsweise solche, die zu den eingangs als besonders wertvoll bezeichneten Verbindungen führen.

Die pharmazeutischen Präparate der vorliegenden Erfindung enthaltend eine Verbindung der Formel I können zur Behandlung der oben genannten Indikationen, insbesondere zur Behandlung von benigner Prostata-Hypertrophie verwendet werden. Sie enthalten eine wirksame Menge des Wirkstoffes allein oder im Gemisch mit anorganischen oder organischen, festen oder flüssigen, pharmazeutisch verwendbaren Trägerstoffen und, wenn erwünscht, auch mit anderen pharmakologisch bzw. therapeutisch wertvollen Stoffen, und eignen sich insbesondere zur enteralen, z.B. oralen oder rektalen, zur parenteralen, beispielsweise transdermalen, oder zur topischen Verabreichung.

Die vorliegende Erfindung betrifft insbesondere pharmazeutische Zusammensetzungen enthaltend als Wirkstoff mindestens eine erfindungsgemässe Verbindung der Formel I in Form einer sterilen und/oder isotonischen wässrigen Lösung, oder aber im Gemisch mit mindestens einem festen oder halbfesten Trägerstoff.

Die vorliegende Erfindung betrifft auch Arzneimittel, sowie insbesondere Arzneimittel in Form von Dosierungseinheiten, welche mindestens eine der erfindungsgemässen Verbindungen allein oder im Gemisch mit einem oder mehreren Trägerstoffen enthalten, insbesondere solche in fester Form.

Die Erfindung betrifft insbesondere Arzneimittel in Form von Tabletten (einschliesslich Lutschtabletten, Granulen und Pastillen), Dragées, Kapseln, Pillen, Ampullen, Trockenvials oder Suppositorien enthaltend den oben definierten Wirkstoff allein oder im Gemisch mit einem oder mehreren Trägerstoffen.

Die Trägerstoffe zur Verwendung in den pharmazeutischen Zusammensetzungen (z.B. Granulaten) zur Herstellung von Tabletten, Dragées, Kapseln und Pillen sind z.B. die folgenden:
a) Verdünnungsmittel, z.B. Stärke, Zucker (wie Lactose, Glukose und Saccharose), Mannit, Sorbit und Kieselsäure,
b) Bindemittel, z.B. Carboxymethylcellulose und andere Cellulosederivate, Alginsäure und ihre Salze (wie Natriumalginat), Gelatine, und Polyvinylpyrrolidon,
c) Feuchtigkeitsregulatoren, z.B. Glycerin,
d) Sprengmittel, z.B. Agar-agar, Calciumcarbonat und Natriumbicarbonat,
e) Retardiermittel zur Verlangsamung der Aufnahme des Wirkstoffes, z.B. Paraffin,
f) Beschleuniger der Resorption, z.B. quaternäre Ammoniumverbindungen,
g) oberflächenaktive Mittel, z.B. Cetylalkohol und Glycerinmonostearat,
h) Adsorptionsmittel, z.B. Kaolin und Bentonit,
i) Fliessregulier-. und Schmiermittel, z.B. Talk, Calciumstearat, Magnesiumstearat und feste Polyethylenglykole.

Die Tabletten, Dragées, Kapseln und Pillen enthaltend die obengenannten erfindungsgemässen pharmazeutischen Zusammensetzungen können mit den üblichen Ueberzügen und Mantelmaterialien versehen werden, denen gewünschtenfalls Farbstoffe oder Pigmente, z.B. zur Identifizierungs- oder Kennzeichnungszwecken, beigemischt werden. Diese Ueberzüge können auch eine solche Zusammensetzung haben, welche eine verlangsamte Abgabe des Wirkstoffes ermöglicht; zu diesem Zwecke sind z.B. Wachse und Cellulosepräparate, wie Acetylcellulosephthalat, oder Hydroxypropylmethylcellulosephthalat, geeignet.

Diese Zusammensetzungen können auch in die Form von Mikrokapseln verarbeitet werden.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichungen eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Eine Zusammensetzung für die topische Applikation kann in Form einer wässrigen Lösung, eines Gels, einer öligen Lösung oder Suspension oder einer fetthaltigen oder insbesondere emulgierten Salbe vorliegen. Eine Zusammensetzung in Form einer wässrigen Lösung gewinnt man beispielsweise durch Lösen der erfindungsgemässen Wirkstoffe in einer wässrigen Pufferlösung mit einem pH 4 bis 6,5, und, wenn gewünscht, der Zugabe eines weiteren Wirkstoffes, beispielsweise eines antiinflammatorischen Mittels, und/oder eines polymeren Bindemittels, beispielsweise Polyvinylpyrrolidon, und/oder eines Schutzmittels. Die Konzentration des Wirkstoffes liegt zwischen etwa 0,1 und etwa 1,5 mg, vorzugsweise zwischen 0,25 und 1,0 mg in 10 ml Lösung oder 10 g Gel.

Eine ölige Verabreichungsform für topische Auftragung wird gewonnen, wenn beispielsweise ein erfindungsgemässer Wirkstoff in einem Öl, wahlweise unter Zusatz von Quellungsmitteln wie Aluminiumstearat, und/oder oberflächenaktiven Stoffen (Tensiden), die einen HLB-Wert unter 10 haben wie Fettsäuremonoester von Polyolen, zum Beispiel Glycerinmonostearat, Sorbitanmonolaurat, Sorbitanmonostearat oder Sorbitanmonooleat, suspendiert werden. Eine fetthaltige Salbe erhält man beispielsweise, wenn ein erfindungsgemässer Wirkstoff oder ein Salz davon in einer streichfähigen Fettgrundlage, wahlweise unter Zusatz eines Tensids mit einem HLB-Wert unter 10 suspendiert wird. Eine emulgierte Salbe erhält man durch Trituration einer wässrigen Lösung des erfindungsgemässen Wirkstoffes in einer weichen streichfähigen Grundlage unter Zusatz eines Tensids mit einem HLB-Wert unter 10. Alle diese Formen für die topische Auftragung können auch Schutzmittel enthalten. Die Konzentration des Wirkstoffes liegt zwischen etwa 0,1 und etwa 1,5 mg, vorzugsweise zwischen 0,25 und 1,0 mg in einer Grundlage von etwa 10 g.

Die erfindungsgemässen pharmazeutischen Zusammensetzungen enthalten vorzugsweise von etwa 0,1 bis zu etwa 99,5, insbesondere von etwa 1 bis zu etwa 90 Gewichts-% des Wirkstoffes.

Die Erfindung betrifft ebenfalls ein Verfahren zur Behandlung der oben genannten Krankheitszustände, wobei man die erfindungsgemässen Verbindungen vorzugsweise in Form von pharmazeutischen Präparaten verwendet. Dabei wird bei einem Körpergewicht von 70 kg eine tägliche Dosis von etwa 1 mg bis etwa 100 mg bei parenteraler oder enteraler Applikation gegeben.

Die Herstellung der obengenannten erfindungsgemässen pharmazeutischen Zusammensetzungen und Arzneimittel erfolgt mittels konventioneller, an sich bekannter Herstellungsverfahren der pharmazeutischen Industrie, z.B. mittels üblicher Misch-, Granulier-, Tablettier-, Dragier-, Lösungs- und Lyophilisierungsverfahren, wobei gewünschtenfalls unter keimfreien Bedingungen gearbeitet wird oder ein Zwischenprodukt oder ein fertiges Produkt sterilisiert wird.

In den folgenden Beispielen, welche die Erfindung illustrieren, ohne sie dabei einzuschränken, sind die Temperaturen in Celsiusgraden angegeben. Alle Schmelzpunkte sind unkorrigiert. Der Drehwinkel [α]_{D} wird bei einer Temperatur von 20° gemessen.

### Beispiel 1: (2-Cyanethyl) 3-Oxo-4aza-5α-androstan-17β-carboxylat

Eine Suspension von 638 mg (2 mmol) 3-Oxo-4-aza-5α-androstan- 17β-carbonsäure in 30 ml Chloroform (frisch über basischem Aluminiumoxid filtriert) wird unter Rühren in Argon-Atmosphäre bei Raumtemperatur mit 1,2 ml 1-Chlor-1-dimethylamino-2-methylprop-1-en in 6 ml Chloroform versetzt und zwei Stunden weitergerührt. Die entstandene klare Lösung, welche 3-Oxo-4-aza-5α-androstan-17β-carbonsäurechlorid enthält, wird unter Rühren bei 4° zu einer Lösung von 1,2 ml 3-Hydroxypropionitril in 6 ml Chloroform zugetropft und 2 Stunden bei Raumtemperatur gerührt. Das Reaktionsgemisch wird mit 5 ml Eiswasser verdünnt und zweimal mit Methylenchlorid extrahiert. Die organischen Phasen werden mit gesättigter Kochsalz-Lösung gewaschen, getrocknet und eingedampft. Durch Chromatographie des Rückstandes über 25 g Kieselgel mit einem Gemisch von Toluol-Aceton (4:1) erhält man (2-Cyanethyl) 3-Oxo-4-aza-5α-androstan-17β -carboxylat, welches nach Kristallisation aus Methylenchlorid-Diisopropylether bei 233-235° schmiltzt, [α]_{D} = + 55.8° (c = 0,554 in Chloroform).

### Beispiel 2: N-(5-Cyanpentyl)-3-oxo-4-aza-5α-androstan-17β-carboxamid

Eine Suspension von 957 mg (3 mmol) 3-Oxo-4-aza-5α-androstan-17β-carbonsäure in 6,6 ml Dimethylformamid wird mit 1.59 g Benztriazol-1-yl-oxy-tris(dimethylamino)phosphonium-hexafluorophosphat (Castros Reagens), 0,6 ml Methylmorpholin, 2,3 ml 6-Aminocapronitril versetzt und 2 Stunden bei Raumtemperatur gerührt. Die entstandene Lösung wird mit Chloroform verdünnt und nacheinander mit Natriumhydrogencarbonat-Lösung , Wasser und gesättigter Kochsalz-Lösung gewaschen. Die organische Phase wird mit Natriumsulfat getrocknet und am Wasserstrahlvakuum zur Trockne eingeengt. Der Rückstand wird über eine Kieselgelsäule chromatographiert. Mit einem Gemisch Toluol-Methanol (97:3) eluiert man N-(5-Cyanpentyl)-3-oxo-4-aza-5α -androstan-17β-carboxamid, Smp 213-215°, [α]_{D} =+ 41.0° (c = 0,488 in Chloroform) nach Kristallisation aus Methylenchlorid-Diisopropylether.

### Beispiel 3: 3-Cyan-2-methyl-2-propyl-3-Oxo-4-aza-5α-androstan-17β-carboxylat

In analoger Weise wie in Beispiel 1 beschrieben, wird 3-Oxo-4-aza-5α-androstan-17β -carbonsäurechlorid mit 3-Hydroxy-3-methylbutyronitril in Chloroform umgesetzt und weiterverarbeitet. Nach Chromatographie an Kieselgel mit einem Gemisch von Toluol-Methanol (98:2) erhält man 3-Cyan-2-methyl-2-propyl 3-Oxo-4-aza-5α -androstan- 17β-carboxylat, welches nach Kristallisation aus Methylenchlorid-Diisopropylether bei 225-227° schmilzt, [α]_{D} = + 59.6° (c = 0,534 in Chloroform).

### Beispiel 4: N-(4-Cyanphenyl)-3-oxo-4-aza-5α-androstan-17β-carboxamid

In analoger Weise wie im Beispiel 1 beschrieben wird das entstandene 3-Oxo-4-aza-5α-androstan-17β-carbonsäurechlorid mit 4-Aminobenzonitril in Chloroform umgesetzt und weiterverarbeitet. Dabei wird N-(4-Cyanphenyl)-3-oxo-4-aza-5α-androstan-17β-carboxamid erhalten, Smp. > 330°, [α]_{D}= + 120,8° (c = 0.582 in Chloroform-Methanol (1:1)).

### Beispiel 5: N-(2-Cyan-2-propyl)-3-oxo-4-aza-5α-androstan-17β-carboxamid

In Analogie zu dem in Beispiel 1 beschriebenen Verfahren wird 3-Oxo-4-aza-5α -androstan-17β-carbonsäurechlorid mit 2-Amino-2-methyl-propionitril in Chloroform umgesetzt und weiterverarbeitet, wobei N-(2-Cyan-2-propyl)-3-oxo-4-aza-5α-androstan-17β-carboxamid, Smp. 304-306°, [α]_{D} = + 83.4° (c = 0,519 in Chloroform-Methanol (1:1)), erhalten wird.

### Beispiel 6: 2-Cyanethyl 3-Oxo-4-aza-5α-androst-1-en-17β-carboxylat

372 mg (1 mmol) (2-Cyanethyl) 3-Oxo-4-aza-5α-androstan-17β-carboxylat, 1,4 ml N,O-Bis-(trimethylsilyl)-trifluoracetamid und 238 mg 2,3-Dichlor-5,6-dicyan- 1,4-benzochinon werden in 8 ml abs. Dioxan suspendiert und unter Argon zuerst 4 Stunden bei 25° gerührt und anschliessend 16 Stunden am Rückfluss erwärmt. Die während der Umsetzung entstehende dunkelrote Lösung wird anschliessend auf die Hälfte eingeengt. Zur Aufarbeitung wird der Rückstand mit 30 ml Methylenchlorid verdünnt und nacheinander mit 1 %-iger Natriumbisulfit-Lösung und gesättigter wässriger Kochsalz-Lösung gewaschen. Die wässrigen Anteile werden mit Methylenchlorid nachextrahiert, die vereinigten organischen Anteile über Natriumsulfat getrocknet und eingedampft. Das so erhaltene Rohprodukt liefert nach Chromatographie über Kieselgel (Toluol-Aceton = 9:1) und anschliessender Kristallisation der einheitlichen Fraktionen aus Methylenchlorid-Diisopropylether das reine 2-Cyanethyl 3-Oxo-4-aza-5α-androst- 1-en-17β -carboxylat mit einem Schmelzpunkt von 259-261°, [α]_{D}= + 2.9° (c = 0,481 in Chloroform).

### Beispiel 7: 3-Cyan-2-methyl-2-propyl 3-Oxo-4-aza-5α-androst-1-en-17β-carboxylat

Analog Beispiel 6 wird 3-Cyan-2-methyl-2-propyl 3-Oxo-4-aza-5α-androstan-17β -carboxylat mit N,O-Bis-(trimethylsilyl)-trifluoracetamid und 2,3-Dicyano-5,6-dichlorbenzochinon in Dioxan umgesetzt und weiterverarbeitet. Dabei wird 3-Cyan-2-methyl-2-propyl 3-Oxo-4-aza-5a-androst-1-en-17β-carboxylat erhalten, Smp. 211-213°, [α]_{D} = + 6.5° (c = 0,428 in Chloroform).

### Beispiel 8: N-(5-Cyanpentyl)-3-oxo-4-aza-5α-androst-1-en-17β-carboxamid

In analoger Weise wie in Beispiel 6 wird N-(5-Cyanpentyl)-3-oxo-4-aza-5α-androstan-17ß-carboxamid mit N,O-Bis-(trimethylsilyl)-trifluoracetamid und 2,3-Dichlor-5,6-dicyan- 1,4-benzochinon umgesetzt und weiterverarbeitet. Nach der Chromatographie des Rohproduktes an Kieselgel mit einem Gemisch von Methylenchlorid-Aceton (4:1) erhält man N-(5-Cyanpentyl)-3-oxo-4-aza-5α-androst-1-en-17β-carboxamid mit einem Schmelzpunkt von 188-190°, [α]_{D} = - 8.8° (c = 0,433 in Chloroform) nach Kristallisation aus Methylenchlorid-Diisopropylether.

### Beispiel 9: N-(4-Cyanphenyl)-3-oxo-4-aza-5α-androst-1-en-17β-carboxamid

In analoger Weise wie in Beispiel 6 wird N-(4-Cyanphenyl)-4-aza-3-oxo-5α-androstan-17β-carboxamid mit N,O-Bis-(trimethylsilyl)-tfifluoracetamid und 2,3-Dichlor-5,6-dicyan-1,4-benzochinon umgesetzt und weiterverarbeitet. Nach der Chromatographie des Rohproduktes an Kieselgel mit einem Gemisch von Methylenchlorid-Methanol (9:1) erhält man das chromatographisch einheitliche N-(4-Cyanphenyl)-3-oxo-4-aza-5α-androst-1-en-17β-carboxamid mit einem Schmelzpunkt von > 330°, [α]_{D}= + 90.3° (c = 0,404 in Chloroform-Methanol (2:1)) nach Kristallisation aus Methylenchlorid-Diisopropylether.

### Beispiel 10: N-(2-Cyan-2-propyl)-3-oxo-4-aza-5α-androst-1-en-17β-carboxamid,

In analoger Weise wie in Beispiel 6 wird N-(2-Cyan-2-propyl)-3-oxo-4-aza-3-oxo-5α-androstan-17β-carboxamid mit N,O-Bis-(trimethylsilyl)-trifluoracetamid und 2,3-Dichlor-5,6-dicyan-1,4-benzochinon umgesetzt und weiterverarbeitet Nach Chromatographie des Rohproduktes mit einem Gemisch von Methylenchlorid-Aceton (4:1) über Kieselgel erhält man das N-(2-Cyan-2-propyl)-3-oxo-4-aza-5α-androst-1-en-17β-carboxamid, das nach einmaligem Umlösen aus Methylenchlorid-Diisopropylether bei 289-291° schmilzt, [α]_{D} = + 14.3° (c = 0,384 in Chloroform).

### Beispiel 11: N-(2-Cyan-2-propyl)-3-oxo-4-aza-5α-androst-1-en-17β-carboxamid

951 mg (3 mmol) 3-Oxo-4-aza-5α-androst-1-en-17β-carbonsäure (J. Am. Chem. Soc. 110, 3318 (1988)) werden in 50 ml Chloroform suspendiert und bei 10 bis 15°C innerhalb von 5 Minuten mit 4,8 ml Thionylchlorid in 21 ml Chloroform versetzt. Die entstandene Lösung wird eine Stunde gerührt. Das Reaktionsgemisch wird anschliessend am Hochvakuum zur Trockne eingeengt. Der Rückstand, welcher 3-Oxo-4-aza-5α-androst-1-en-17β-carbonsäurechlorid enthält, wird in 60 ml Chloroform gelöst, bei 25° unter Rühren tropfenweise nacheinander mit 0,2 ml Triethylamin und 1,95 ml 2-Amino-2-methylpropionitril in 18 ml Chloroform versetzt und bei Raumtemperatur 3 Stunden gerührt. Das Reaktionsgemisch wird mit 60 ml Eiswasser verdünnt und mit Methylenchlorid extrahiert Die organische Phase wird mit gesättigter Kochsalzlösung gewaschen, getrocknet und im Wasserstrahlvakuum eingeengt. Der Rückstand wird an einer Kieselgelsäule chromatographiert. Mit einem Gemisch von Methylenchlorid-Aceton (9:1) eluiert man N-(2-Cyan-2-propyl)-3-oxo-4-aza-5α-androst-1-en-17β-carboxamid, das nach Kristallisation aus Methylenchlorid-Diisopropylether bei 291-295° schmilzt.

### Beispiel 12: N-(2-Cyan-2-propyl)-4-methyl-3-oxo-4-aza-5α-androstan-17β-carboxamid

Analog zu dem in Beispiel 11 beschriebenen Verfahren wird N-(2-Cyan-2-propyl)-4-methyl-3-oxo-4-aza-5α-androstan-17β-carboxamid aus 4-Methyl-3-oxo-4-aza-5α-androstan-17β-carbonsäure (J. Med. Chem. 27, 1690(1984)) hergestellt, das nach Kristallisation aus Methylenchlorid-Diisopropylether bei 233-235°schmilzt; [α]_{D}=+49.3°(c=0,475 in Chloroform).

### Beispiel 13: N-(2-Cyan-2-propyl)-4-methyl-3-oxo-4-aza-5α-androst-1-en-17β-carboxamid

Analog zu dem in Beispiel 11 beschriebenen Verfahren erhält man aus 4-Methyl-3-oxo-4-aza-5α-androst-1-en-17β-carbonsäure (J. Med. Chem. 29, 2298 (1986)) N-(2-Cyan-2-propyl)-4-methyl-3-oxo-4-aza-5α-androst-1-en-17β-carboxamid, das nach Umkristallisation aus Methylenchlorid-Diisopropylether bei 231-235° schmilzt; [α]_{D}= + 3.8° (c = 0,44 in Chloroform).

### Beispiel 14: N-(3-Cyan-3-pentyl)-3-oxo-4-aza-5α-androst-1-en-17β-carboxamid

In analoger Weise wie in Beispiel 11 beschrieben, wird das entstandene 3-oxo-4-aza-5α-androst -1-en- 17β-carbonsäurechlorid mit 2-Amino-2-ethyl-butyronitril in Chloroform umgesetzt und weiterverarbeitet. Dabei wird N-(3-Cyan-3-pentyl)-3-oxo-4-aza-5α -androst-1-en- 17β-carboxamid erhalten, Smp. 280-288°, [α]_{D}= 13.7° (c = 0.467 in Chloroform).

### Beispiel 15: N-(2-Cyan-2-butyl)-3-oxo-4-aza-5α-androst-1-en-17β-carboxamid

In analoger Weise wie in Beispiel 11 beschrieben, wird das entstandene 3-Oxo-4-aza-5α-androst -1-en-17β-carbonsäurechlorid mit 2-Amino-2-methyl-butyronitril in Chloroform umgesetzt und weiterverarbeitet. Dabei wird N-(2-Cyan-2-butyl)-3-oxo-4-aza-5α-androst-1-en 17β-carboxamid erhalten, Smp. 262-265°.

### Beispiel 16: N-(1-Cyan-1-cyclopropyl)-3-oxo-4-aza-5α-androst-1-en-17β-carboxamid

In analoger Weise wie in Beispiel 11 beschrieben, wird das entstandene getrocknete 3-Oxo-4-aza-5α-androst-1-en-17β-carbonsäurechlorid (1 mmol) in 12 ml Chloroform gelöst, bei 25°C unter Rühren tropfenweise mit 0,3 ml Triethylamin und einer Suspension aus 472 mg (4 mmol) 1-Amino-1-cyclopropancarbonitrilhydrochlorid (J. Org. Chem. 55, 4281 (1990)) und 0,73 ml N-Ethyldiisopropylamin umgesetzt und weiterverarbeitet. Dabei wird N-(1-Cyan-1-cyclopropyl)-3-oxo-4-aza-5α-androst-1-en-17β-carboxamid erhalten.

### Beispiel 17: N-(1-Cyan-3-cyclopentyl)-3-oxo-4-aza-5α-androst-1-en-17β-Carboxamid

In analoger Weise wie in Beispiel 11 beschrieben, wird das entstandene 3-Oxo-4-aza-5α-androst -1-en-17β-carbonsäurechlorid mit 1-Amino-1-cyclopentancarbonitril in Chloroform umgesetzt und weiterverarbeitet. Dabei wird N-(1-Cyan-1-cyclopentyl)-3-oxo-5α-androst-1-en-17β-carboxamid erhalten, Smp. 262-269°, [α]_{D}= +18.8° (c = 0,54 in Chloroform).

### Beispiel 18: N-(1-Cyan-1-cyclohexyl)-3-oxo-4-aza-5α-androst-1-en-17β-carboxamid

In analoger Weise wie in Beispiel 11 beschrieben, wird das entstandene 3-Oxo-4-aza-5α-androst -1-en-17β-carbonsäurechlorid mit 1-Amino-1-cyclohexancarbonitril in Chloroform umgesetzt und weiterverarbeitet. Dabei wird N-(1-Cyan-1-cyclohexyl)-3-oxo-5α-androst-1-en-17β-carboxamid erhalten, Smp. 292-300°, [α]_{D}= +18.7° (c=0,486 in Chloroform).

### Beispiel 19: N-(2-Cyanphenyl)-3-oxo-4-aza-5α-androst-1-en-17β-carboxamid

In analoger Weise wie in Beispiel 11 beschrieben, wird 3-Oxo-4-aza-5α-androst-1-en-17β -carbonsäurechlorid mit 2-Aminobenzonitril in Chloroform umgesetzt und weiterverarbeitet, wobei N-(2-Cyanphenyl)-3-oxo-4-aza-5α-androst-1-en-17β-carboxamid, Smp. 296-304°, erhalten wird.

### Beispiel 20: N-(3-Cyanphenyl)-3-oxo-4-aza-5α-androstan-17β-carboxamid

In Analogie zu dem im Beispiel 1 beschriebenen Verfahren wird 4-Aza-3-oxo-5α-androstan 17β-carbonsäurechlorid mit 3-Aminobenzonitril in Tetrahydrofuran umgesetzt und weiterverarbeitet. Dabei wird N-(3-Cyanphenyl)-3-oxo-4-aza-5α-androstan-17β-carboxamid erhalten, Smp. 299-301°.

### Beispiel 21: N-(2-Cyanphenyl)-3-oxo-4-aza-5α-androstan-17β-carboxamid

In Analogie zu dem im Beispiel 1 beschriebenen Verfahren wird das 4-Aza-3-oxo-5α-androstan 17β-carbonsäurechlorid mit 3-Aminobenzonitril in Chloroform umgesetzt und weiterverarbeitet. Dabei wird N-(2-Cyanphenyl)-3-oxo-4-aza-5α-androstan-17β-carboxamid erhalten, Smp. 298-306°.

### Beispiel 22: N-(3-Cyanphenyl)-3-oxo-4-aza-5α-androst-1-en-17β-carboxamid

In analoger Weise wie in Beispiel 6 beschrieben, wird N-(3-Cyanphenyl)-3-oxo-4-aza-5α-androstan-17β-carboxamid mit N,O-Bis-(trimethylsilyl)-trifluoracetamid und 2,3-Dichlor-5,6-dicyan-1,4-benzochinon umgesetzt und weiterverarbeitet. Nach der Chromatographie des Rohproduktes an Kieselgel mit einem Gemisch von Methylenchlorid-Aceton (4:1) erhält man N-(3-Cyanphenyl)-3-oxo-4-aza-5α-androst-1-en-17β-carboxamid, das nach Kristallisation aus Methylenchlorid-Diisopropylether bei 318-321° schmilzt.

### Beispiel 23: N,N-(Methyl)(2-Cyan-2-propyl)-4-methyl-3-oxo-4-aza-5α-androst-1-en-17β-carboxamid

Eine Lösung von 144 mg (0.3 mmol) N-(2-Cyan-2-propyl)-3-oxo-4-aza-5α-androst-1 -en-17β-carboxamid in 3.0 ml abs. Tetrahydrofuran wird mit 0,4 ml Methyljodid und 80 mg Natriumhydrid Dispersion versetzt und bei Raumtemperatur 5 Stunden unter Argon-Atmosphäre gerührt. Zur Aufarbeitung wird das Reaktionsgemisch zu 5 ml Eiswasser und 1 ml Essigsäure gegossen und zweimal mit Methylenchlorid extrahiert. Die organischen Phasen werden mit gesättigter Kochsalzlösung gewaschen, getrocknet und eingedampft. Durch Chromatographie des Rückstandes über Kieselgel mit dem Gemisch von Methylenchlorid-Aceton (95:5) erhält man N,N-(Methyl)(2-Cyan-2-propyl)-4-methyl-3-oxo-4-aza-5α-androst-1-en-17β-carboxamid, welches nach Kristallisation aus Methylenchlorid-Diisopropylether bei 218-221° schmilzt; [α]_{D}= +25.8° (c = 0,485 in Chloroform).

### Beispiel 24:

Tabletten, enthaltend 10 mg Wirkstoff, z.B. N-(2-Cyan-2-propyl)-3-oxo-4-aza-5α-androst-1-en-17β-carboxamid oder eine andere Verbindung der Beispiele 1-23 werden wie folgt hergestellt:

| Zusammensetzung für 5000 Tabletten | |
|---|---|
| Wirkstoff, feinst gemahlen | 50,0 g |
| Saccharose | 79,0 g |
| Arabisches Gummi | 4,75 g |
| Sorbit | 3,75 g |
| Talk | 2,5 g |
| Magnesiumstearat | 4,9 g |
| Mineralöl | 0,1 g |
| Carboxymethylcellulose (Na-Salz) | 5,0 g |

Herstellung: Der Wirkstoff wird mit gepuderter Saccharose und dem arabischen Gummi vermischt, gesiebt und mittels einer etwa 35-prozentigen wässrigen Sorbit-Lösung granuliert. Das Granulat wird durch ein Sieb getrieben, getrocknet, nochmals gesiebt und mit den übrigen Hilfsstoffen (Talk, Magnesiumstearat, Mineralöl und Carboxymethylcellulose-Natriumsalz) innig vermischt. Die Mischung wird in üblicher Weise zu Tabletten von 10 mg verpresst.

### Beispiel 25:

Tabletten, enthaltend 1 mg Wirkstoff, z.B. N-(2-Cyan-2-propyl)-3-oxo-4-aza-5α-androst-1-en-17β-carboxamid oder eine andere Verbindung der Beispiele 1-23 werden wie folgt hergestellt:

| Zusammensetzung für 5000 Tabletten | |
|---|---|
| Wirkstoff, feinst gemahlen | 50,0 g |
| Saccharose | 79,0 g |
| Arabisches Gummi | 4,75 g |
| Sorbit | 3,75 g |
| Talk | 2,5 g |
| Magnesiumstearat | 4,9 g |
| Mineralöl | 0,1 g |
| Carboxymethylcellulose (Na-Salz) | 5,0 g |

Herstellung: Der Wirkstoff wird mit gepuderter Saccharose und dem arabischen Gummi vermischt, gesiebt und mittels einer etwa 35-prozentigen wässrigen Sorbit-Lösung granuliert. Das Granulat wird durch ein Sieb getrieben, getrocknet, nochmals gesiebt und mit den übrigen Hilfsstoffen (Talk, Magnesiumstearat, Mineralöl und Carboxymethylcellulose-Natriumsalz) innig vermischt. Die Mischung wird in üblicher Weise zu Tabletten von 1 mg verpresst.

## Patentansprüche

1. Verbindungen der Formel 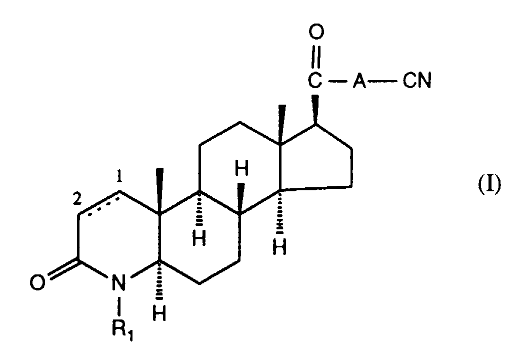 worin die Kohlenstoffatome 1 und 2 durch eine Einfachbindung oder eine Doppelbindung verbunden sind, R₁ Wasserstoff, Methyl oder Ethyl bedeutet, und A für eine Gruppe der Formel -N(-R₂)-X-, worin R₂ Wasserstoff oder C₁-C₄-Alkyl, und X C₁-C₁₂-Alkylen oder C₃-C₆- Cycloalkyliden bedeutet; eine Gruppe der Formel -N(-R₂)-Y-Phe-, worin R₂ die oben genannte Bedeutung hat, Y eine direkte Bindung oder C₁-C₆-Alkylen ist, und Phe einen gegebenenfalls substituierten Phenylenrest bezeichnet; eine Gruppe der Formel -O-X-, worin X die oben genannte Bedeutung hat, oder eine Gruppe -O-Y-Phe- steht, worin Y und Phe die oben genannten Bedeutungen haben.

2. Verbindungen der Formel I gemäss Anspruch 1, worin die Kohlenstoffatome 1 und 2 durch eine Einfachbindung oder eine Doppelbindung verbunden sind, R₁ Wasserstoff oder Methyl bedeutet, und A für eine Gruppe der Formel -N(-R₂)-X-, worin R₂ Wasserstoff und X geradkettiges oder verzweigtes C₁-C₆ Alkylen bedeutet; eine Gruppe der Formel -N(-R₂)-Y-Phe-, worin R₂ die oben genannte Bedeutung hat, Y eine direkte Bindung oder C₁-C₄-Alkylen ist, und Phe einen Phenylenrest bezeichnet, der gegebenenfalls durch Halogen, C₁-C₄-Alkyl, C₁-C₄-Alkoxy, Hydroxy, Nitro, Cyano oder C₁-C₄-Alkoxycarbonyl substituiert ist; eine Gruppe der Formel -O-X-, worin X die oben genannte Bedeutung hat, oder eine Gruppe -O-Y-Phe- steht, wobei Y und Phe die oben genannten Bedeutungen haben.

3. Verbindungen der Formel I gemäss Anspruch 1, worin die Kohlenstoffatome 1 und 2 durch eine Doppelbindung verbunden sind, R₁ Wasserstoff bedeutet, und A für eine Gruppe der Formel -N(-R₂)-X-, worin R₂ Wasserstoff und X C₁-C₄ Alkylen bedeutet; oder eine Gruppe der Formel -N(-R₂)-Y-Phe-, worin R₂ die oben genannte Bedeutung hat, Y eine direkte Bindung ist, und Phe einen p-Phenylenrest bezeichnet.

4. Verbindungen der Formel I gemäss Anspruch 1, worin die Kohlenstoffatome 1 und 2 durch eine Einfach- oder eine Doppelbindung verbunden sind, und A für eine Gruppe der Formel -N(-R₂)-Y-Phe- steht, worin R₂ Wasserstoff, Y eine direkte Bindung und Phe ein p-Phenylenrest ist.

5. Verbindungen der Formel I gemäss Anspruch 1, worin die Kohlenstoffatome 1 und 2 durch eine Doppelbindung verbunden sind, R₁ Wasserstoff bedeutet, und A für eine Gruppe der Formel -N(-R₂)-X- steht, worin R₂ Wasserstoff und X 1,1-Cyclopentyliden oder 1,1-Cyclhexyliden bedeutet.

6. N-(4-Cyanphenyl)-3-oxo-4-aza-5α-androst-1-en-17β-carboxamid gemäss Abspruch 4.

7. N-(2-Cyan-2-propyl)-3-oxo-4-aza-5α-androst-1-en-17β-carboxamid gemäss Anspruch 1.

8. N-(2-Cyan-2-propyl)-4-methyl-3-oxo-4-aza-5α-androstan-17β-carboxamid gemäss Anspruch 1.

9. Pharmazeutische Zusammensetzung enthaltend eine Verbindung der Formel I gemäss Anspruch 1.

10. Verwendung einer Verbindung der Formel I gemäss Anspruch 1 zur Anwendung in einem Verfahren zur therapeutischen Behandlung des menschlichen oder tierischen Körpers. Verfahren zur Herstellung von Verbindungen der Formel I gemäss Anspruch 1, dadurch gekennzeichnet, dass man
(a) eine Verbindung der Formel 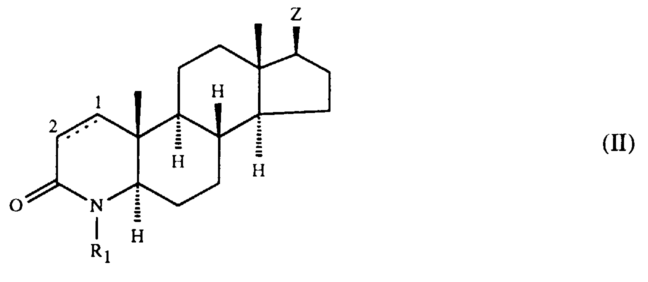 worin die Kohlenstoffatome 1 und 2 durch eine Einfachbindung oder eine Doppelbindung verbunden sind, R₁ die unter Formel I angegebene Bedeutung hat, und Z für Carboxyl oder eine reaktionsfähig aktivierte Carboxylgruppe steht, mit einer Verbindung der Formel H-A₁-CN, worin A₁ für eine Gruppe der Formel -N(-R₂)-X- oder für eine Gruppe der Formel -N(-R₂)-Y-Phe- steht, oder mit einer Verbindung der Formel H-A₂-CN umsetzt, worin A₂ für eine Gruppe der Formel -O-X- oder für eine Gruppe der Formel -O-Y-Phe-steht, worin R₂, X, Y und Phe die unter Formel I angegebenen Bedeutungen haben, oder
(b) in einer Verbindung der Formel 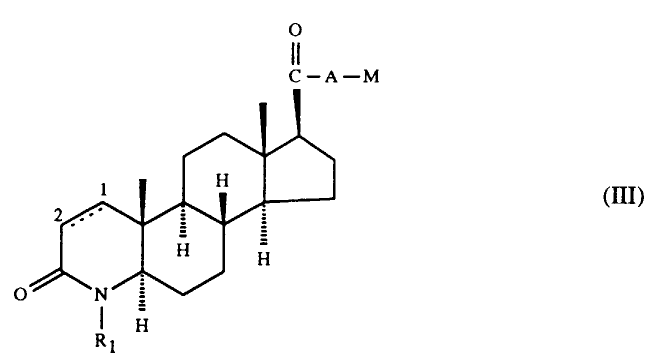 worin die Kohlenstoffatome 1 und 2 durch eine Einfachbindung oder eine Doppelbindung verbunden sind, R₁ und A wie unter Formel I definiert sind, und M einen in Cyano überführbaren Rest bedeutet, den Rest M in Cyano überführt, oder
(c) zur Herstellung einer Verbindung der Formel I, worin die Kohlenstoffatome 1 und 2 durch eine Einfachbindung verbunden sind, und R₁ und A die oben angegebenen Bedeutungen haben, eine Verbindung der Formel 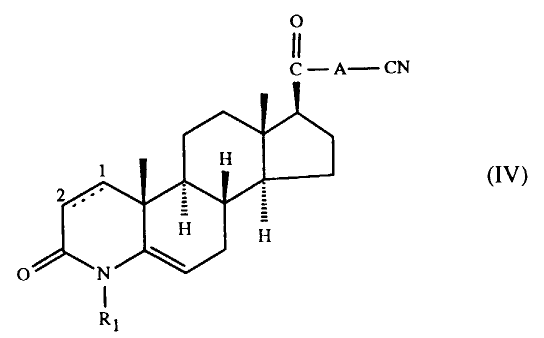 mit einem Reduktionsmittel behandelt, und wenn erwünscht, eine verfahrensgemäss erhältliche Verbindung der Formel I in eine andere Verbindung der Formel I überführt.

11. Verbindungen der Formel I gemäss einem der Ansprüche 1 bis 8 zur Anwendung in einem Verfahren zur therapeutischen Behandlung des menschlichen oder tierischen Körpers.

12. Verwendung einer Verbindung der Formel I gemäss einem der Ansprüche 1 bis 8 zur Herstellung eines Arzneimittels für die therapeutische Behandlung von benigner Prostata-Hypertrophie.

13. Verwendung einer Verbindung der Formel I gemäss einem der Ansprüche 1 bis 8 zur Herstellung eines Arzneimittels für die therapeutische Behandlung von Erkrankungen und Zuständen, die auf eine Reduktion der physiologischen 5α-Dihydrotestosteron Menge günstig ansprechen.

## Claims

1. A compound of the formula 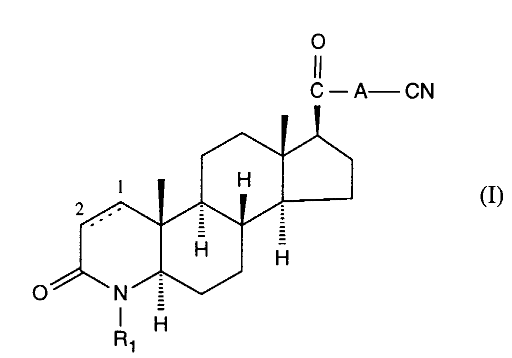 wherein carbon atoms 1 and 2 are linked by a single bond or a double bond, R₁ is hydrogen, methyl or ethyl, and A is a group of the formula -N(-R₂)-X- wherein R₂ is hydrogen or C₁-C₄alkyl and X is C₁-C₁₂alkylene or C₃-C₆cycloalkylidene; a group of the formula -N(-R₂)-Y-Phe- wherein R₂ is as defined above, Y is a direct bond or C₁-C₆-alkylene and Phe is an unsubstituted or substituted phenylene radical; a group of the formula -O-X- wherein X is as defined above, or a group -O-Y-Phe- wherein Y and Phe are as defined above.

2. A compound of formula I according to claim 1 wherein carbon atoms 1 and 2 are linked by a single bond or a double bond, R₁ is hydrogen or methyl, and A is a group of the formula -N(-R₂)-X- wherein R₂ is hydrogen and X is straight-chain or branched C₁-C₆-alkylene; a group of the formula -N(-R₂)-Y-Phe- wherein R₂ is as defined above, Y is a direct bond or C₁-C₄alkylene and Phe is a phenylene radical that is unsubstituted or substituted by halogen, C₁-C₄alkyl, C₁-C₄alkoxy, hydroxy, nitro, cyano or by C₁-C₄alkoxycarbonyl; a group of the formula -O-X- wherein X is as defined above, or a group -O-Y-Phe- wherein Y and Phe are as defined above.

3. A compound of formula I according to claim 1 wherein carbon atoms 1 and 2 are linked by a double bond, R₁ is hydrogen, and A is a group of the formula -N(-R₂)-X- wherein R₂ is hydrogen and X is C₁-C₄alkylene; or a group of the formula -N(-R₂)-Y-Phe- wherein R₂ is as defined above, Y is a direct bond and Phe is a p-phenylene radical.

4. A compound of formula I according to claim 1 wherein carbon atoms 1 and 2 are linked by a single or a double bond, and A is a group of the formula -N(-R₂)-Y-Phe- wherein R₂ is hydrogen, Y is a direct bond and Phe is a p-phenylene radical.

5. A compound of formula I according to claim 1 wherein carbon atoms 1 and 2 are linked by a double bond, R₁ is hydrogen and A is a group of the formula -N(-R₂)-X- wherein R₂ is hydrogen and X is 1,1-cyclopentylidene or 1,1-cyclohexylidene.

6. N-(4-Cyanophenyl)-3-oxo-4-aza-5α-androst-1-ene-17β-carboxamide according to claim 4.

7. N-(2-Cyano-2-propyl)-3-oxo-4-aza-5α-androst-1-ene-17β-carboxamide according to claim 1.

8. N-(2-Cyano-2-propyl)-4-methyl-3-oxo-4-aza-5α-androstane-17β-carboxamide according to claim 1.

9. A pharmaceutical composition comprising a compound of formula I according to claim 1.

10. A process for the preparation of a compound of formula I according to claim 1, which process comprises
(a) reacting a compound of formula 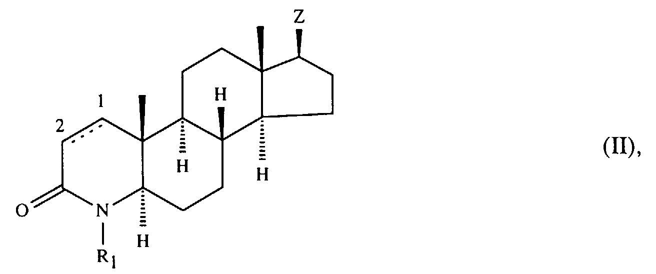 wherein carbon atoms 1 and 2 are linked by a single bond or a double bond, R₁ is as defined for formula I, and Z is carboxy or a reactively activated carboxy group, with a compound of the formula H-A₁-CN wherein A₁ is a group of the formula -N(-R₂)-X- or a group of the formula -N(-R₂)-Y-Phe-, or with a compound of the formula H-A₂-CN wherein A₂ is a group of the formula -O-X- or a group of the formula -O-Y-Phe-, wherein R₂, X, Y and Phe are as defined for formula I, or
(b) in a compound of formula 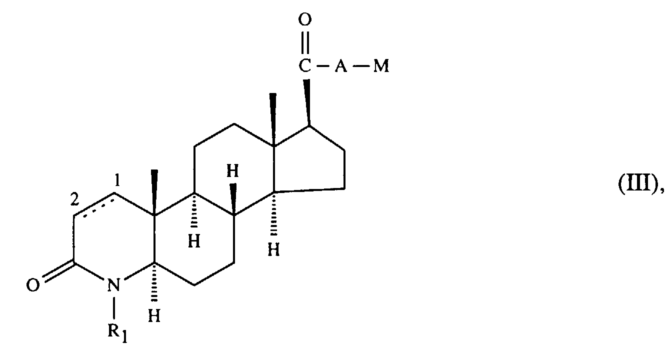 wherein carbon atoms 1 and 2 are linked by a single bond or a double bond, R₁ and A are as defined for formula I, and M is a radical that can be converted into cyano, converting the radical M into cyano, or
(c) for the preparation of a compound of formula I wherein carbon atoms 1 and 2 are linked by a single bond, and R₁ and A are as defined above, treating a compound of formula 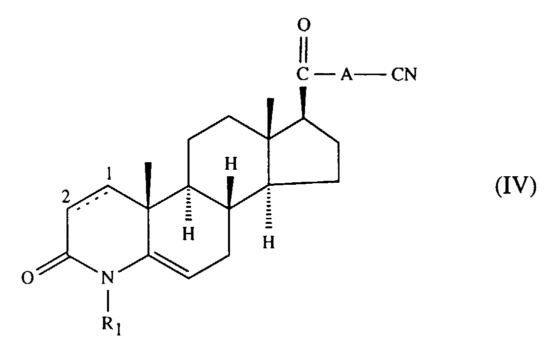 with a reducing agent, and, if desired, converting a compound of formula I obtainable in accordance with the process into a different compound of formula I.

11. A compound of formula I according to any one of claims 1 to 8 for use in a method for the therapeutic treatment of the human or animal body.

12. The use of a compound of formula I according to any one of claims 1 to 8 in the preparation of a medicament for the therapeutic treatment of benign hypertrophy of the prostate.

13. The use of a compound of formula I according to any one of claims 1 to 8 in the preparation of a medicament for the therapeutic treatment of disorders and conditions that respond favourably to a reduction in the amount of physiological 5α-dihydrotestosterone.

## Revendications

1. Composés de formule 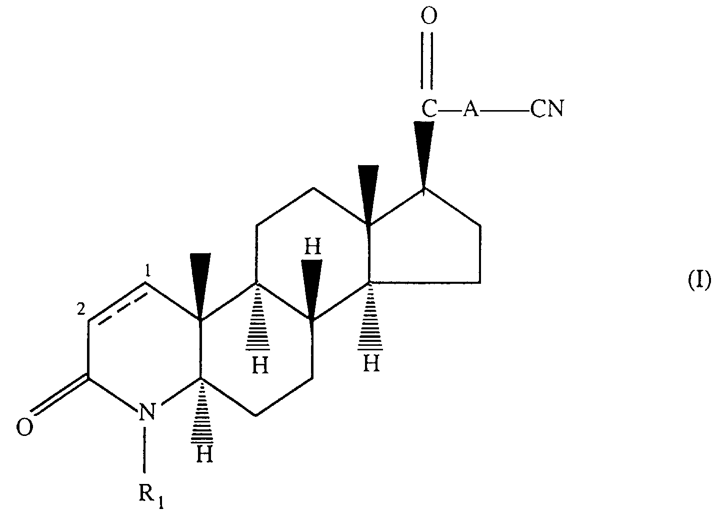 dans laquelle les atomes de carbone 1 et 2 sont reliés par une liaison simple ou double, R₁ représente l'hydrogène, un groupe méthyle ou éthyle et A un groupe de formule -N(-R₂)-X- dans laquelle R₂ représente l'hydrogène ou un groupe alkyle en C₁-C₄ et X représente un groupe alkylène en C₁-C₁₂ ou cycloalkylidène en C₃-C₆ ; un groupe de formule -N(-R₂)-Y-Phe- dans laquelle R₂ a les significations indiquées ci-dessus, Y représente une liaison directe ou un groupe alkylène en C₁-C₆ et Phe un groupe phénylène éventuellement substitué ; un groupe de formule -O-X- dans laquelle X a les significations indiquées ci-dessus, ou un groupe -O-Y-Phe- dans lequel Y et Phe ont les significations indiquées ci-dessus.

2. Composés de formule I selon la revendication 1, dans laquelle les atomes de carbone 1 et 2 sont reliés par une liaison simple ou double, R₁ représente l'hydrogène ou un groupe méthyle et A un groupe de formule -N-(R₂)-X- dans laquelle R₂ représente l'hydrogène et X un groupe alkylène à chaîne droite ou ramifiée en C₁-C₆ ; un groupe de formule -N(-R₂)-Y-Phe- dans laquelle R₂ a les significations indiquées ci-dessus, Y représente une liaison directe ou un groupe alkylène en C₁-C₄ et Phe un groupe phénylène éventuellement substitué par des halogènes, des groupes alkyle en C₁-C₄, alcoxy en C₁-C₄, hydroxy, nitro, cyano ou (alcoxy en C₁-C₄)-carbonyle ; un groupe de formule -O-X- dans laquelle X a les significations indiquées ci-dessus, ou un groupe -O-Y-Phe- dans lequel Y et Phe ont les significations indiquées ci-dessus.

3. Composés de formule I selon la revendication 1, dans laquelle les atomes de carbone 1 et 2 sont reliés par une double liaison, R₁ représente l'hydrogène et A un groupe de formule -N(-R₂)-X- dans laquelle R₂ représente l'hydrogène et X un groupe alkylène en C₁-C₄ ; ou un groupe de formule -N(-R₂)-Y-Phe- dans laquelle R₂ a les significations indiquees ci-dessus, Y représente une liaison directe et Phe un groupe p-phénylène.

4. Composés de formule I selon la revendication 1 dans laquelle les atomes de carbone 1 et 2 sont reliés par une liaison simple ou double et A représente un groupe de formule -N-(-R₂)-Y-phe- dans laquelle R₂ représente l'hydrogène, Y une liaison directe et Phe un groupe p-phénylène.

5. Composés de formule I selon la revendication 1, dans laquelle les atomes de carbone 1 et 2 sont reliés par une double liaison, R₁ représente l'hydrogène et A un groupe de formule -N(-R₂)-X- dans laquelle R₂ représente l'hydrogène et X un groupe 1,1-cyclopentylidène ou 1,1-cyclohexylidène.

6. Le N-(4-cyanophényl)-3-oxo-4-aza-5α-androst-1-ène-17β-carboxamide selon la revendication 4.

7. Le N-(2-cyano-2-propyl)-3-oxo-4-aza-5α-androst-1-ène-1β-carboxamide selon la revendication 1.

8. Le N-(2-cyano-2-propyl)-4-méthyl-3-oxo-4-aza-5α-androstane-1β-carboxamide selon la revendication 1.

9. Composition pharmaceutique contenant un composé de formule I selon la revendication 1.

10. Procédé de préparation des composés de formule I selon la revendication 1, caractérisé en ce que
(a) on fait réagir un composé de formule 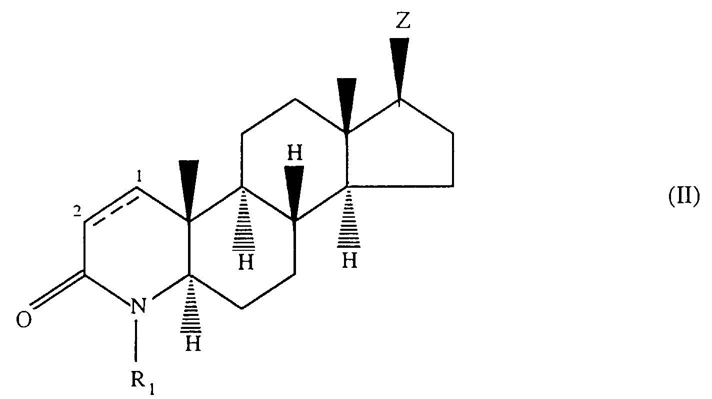 dans laquelle les atomes de carbone 1 et 2 sont reliés par une liaison simple ou double, R₁ a les significations indiquées en référence à la formule I et Z représente un groupe carboxyle éventuellement à l'état activé réactif, avec un composé de formule H-A₁-CN dans laquelle A₁ représente un groupe de formule -N(-R₂)-X- ou un groupe de formule -N(-R₂)-Y-Phe-, ou avec un composé de formule H-A₂-CN dans laquelle A₂ représente un groupe de formule -O-X- ou un groupe de formule -O-Y-Phe- dans lesquelles R₂, X, Y et Phe ont les significations indiquées en référence à la formule I, ou bien
(b) dans un composé de formule 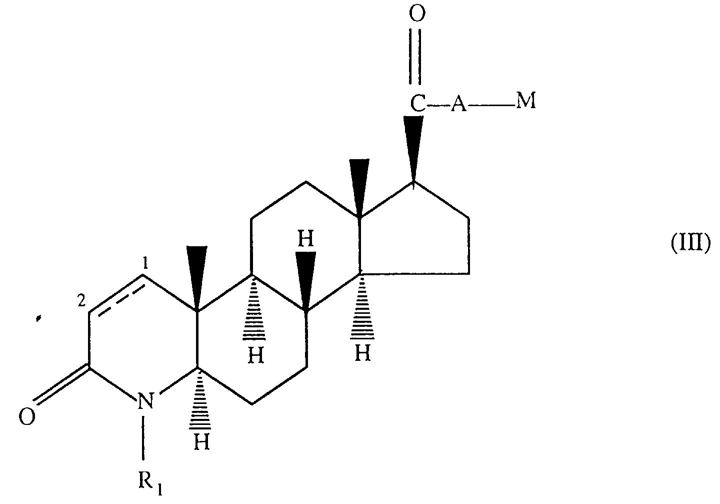 dans laquelle les atomes de carbone 1 et 2 sont reliés par une liaison simple ou double, R₁ et A ont les significations indiquées en référence à la formule I et M représente un groupe convertible en un groupe cyano, on convertit le groupe M en groupe cyano, ou bien
(c) pour préparer un composé de formule I dans laquelle les atomes de carbone 1 et 2 sont reliés par une liaison simple, et R₁ et A ont les significations indiquées ci-dessus, on traite un composé de formule 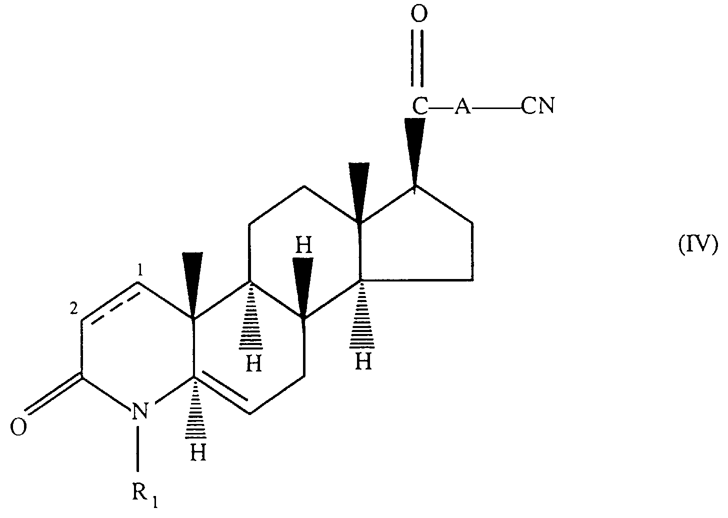 par un agent réducteur, et si on le désire, on convertit un composé de formule I obtenu comme décrit ci-dessus en un autre composé de formule I.

11. Composés de formule I selon l'une des revendications 1 à 8 pour l'utilisation dans un procédé pour le traitement thérapeutique de l'organisme humain ou animal.

12. Utilisation d'un composé de formule I selon l'une des revendications 1 à 8 pour la préparation d'un médicament prévu pour le traitement thérapeutique de l'hypertrophie bénigne de la prostate.

13. Utilisation d'un composé de formule I selon l'une des revendications 1 à 8 pour la préparation d'un médicament prévu pour le traitement thérapeutique des maladies et états réagissant favorablement à une diminution de la quantité physiologique de 5α-dihydrotestostérone.
